**Europäisches Patentamt**

**European Patent Office**

**Office européen des brevets**

(11) Veröffentlichungsnummer: **0 273 174**

**A1**

## EUROPÄISCHE PATENTANMELDUNG

(21) Anmeldenummer: 87116928.0

(22) Anmeldetag: 17.11.87

(51) Int. Cl.4: **C02F 3/10 , C02F 3/06**

(30) Priorität: 05.12.86 DE 3641567

(43) Veröffentlichungstag der Anmeldung:
06.07.88 Patentblatt 88/27

(84) Benannte Vertragsstaaten:
**BE CH FR GB IT LI NL**

(71) Anmelder: **Noell GmbH Wassertechnik Lingen**
**Handelsstrasse 6**
**D-4450 Lingen (Ems)(DE)**

(72) Erfinder: **Menzel, Roman, Dr.-Ing.**
**Quellenweg 10**
**D-4450 Lingen 1(DE)**
Erfinder: **Hoffmann, Peter**
**Meppener Strasse 70i**
**D-4450 Lingen 1(DE)**
Erfinder: **Vorlop, Klaus-Dieter, Dr.**
**Dipl.-Biochem.**
**Hochstrasse 7**
**D-3300 Braunschweig(DE)**

(74) Vertreter: **König, Reimar, Dr.-Ing. et al**
**Patentanwälte Dr.-Ing. Reimar König**
**Dipl.-Ing. Klaus Bergen Wilhelm-Tell-Strasse**
**14 Postfach 260162**
**D-4000 Düsseldorf 1(DE)**

(54) **Verfahren und Vorrichtung zum Entfernen von Ammonium aus Abwasser, Oberflächenwasser oder Grundwasser.**

(57) Verfahren zum Entfernen von Ammonium aus Abwasser, Oberflächenwasser oder Grundwasser mit Hilfe der biologischen Nitrifikation, bei der in einem Reaktorsystem (3, 9) mit Hilfe eines Biokatalysators (4, 12, 19) mit immobilsierten Zellen, die in einem Gel oder Polymer eingeschlossen sind, Ammonium zu Nitrat oxidiert wird. Vorzugsweise werden definierte Hochleistungsmischkulturen von Nitrifikanten (Nitrosomonas, Nitrobacter usw.) verwendet. Zur Erreichung einer hohen Abbaugeschwindigkeit müssen Sauerstoff und Carbonat bzw. $CO_2$ im Reaktorsystem zudosiert werden. Durch die Verwendung der in einem Polymer eingeschlossenen Zellen kann die Reaktorproduktivität gegenüber den klassischen Verfahren um den Faktor 10-20 gesteigert werden.

EP 0 273 174 A1

## "Verfahren und Vorrichtung zum Entfernen von Ammonium aus Abwasser, Oberflächenwasser oder Grundwasser"

Bei vielen industriellen und kommunalen Kläranlagen besteht das Problem, daß die nach dem derzeitigen Stand der Technik gereinigten Abwässer die Vorfluter mit zu hohen $NH_3$ und $NH_4$-Frachten belasten. Die Festlegung von zufriedenstellenden Einleitungsrichtwerten stößt auf die Schwierigkeit, daß zumutbare Prozesse für die Nitrifikation des Abwassers, d.h. für die Umwandlung des Ammoniaks in Nitrat und erforderlichenfalls auch des Nitrats in umweltfreundlichen Stickstoff und Sauerstoff nicht zur Verfügung stehen. Bei den derzeitig bekannten Verfahren zur Behandlung kommunaler und industrieller Abwässer werden durch erhebliche Erhöhung der Verweilzeiten in den Biologien durch geeignete Prozeßführung (mehrstufig) durch den Einsatz von sogenannten Festbetten, die der Bakterienfauna eine Bewuchsfläche anbieten, oder durch Schwimmstoffe, die den Bewuchs der gewünschten Bakterienfauna ermöglichen, verbesserte Ableitungswerte angestrebt. Selbst wenn es gelingt, verweilzeitaufwendige Prozeßtechniken zu entwickeln, stößt der praktische Betrieb später auf die Problematik, daß - schon bei geringfügigen Änderungen der Bedingungen und bei geringfügigen Betreibsstörungen, die biologische Leistung negativ beeinträchtigt wird. Die Ursache ist darin zu suchen, daß die nitrifizierenden Bakterien sehr empfindlich sind und nur sehr langsam wachsen und daß sie nicht oder nur sehr schlecht an Trägermaterialien adsorbieren. Als Folge davon werden sie aus den Reaktoren ausgeschwemmt, so daß die für die schnelle Nitrifikation erforderliche hohe Zelldichte nicht erreicht werden kann. Der Einsatz von Hochleistungsmischkulturen führt bei dieser Art der Anwendung zu keinen positiven Ergebnissen, da die ungeschützten Bakterien durch Fremdverkeimung überwuchert und verdrängt werden.

Neues Verfahren:

In dem neuen Verfahren können erstmals Hochleistungsmischkulturen in Form von eingeschlossenen Zellen eingesetzt werden. In diesen Mischkulturen sind zwei Typen von Zellen vorhanden:

Typ A oxidiert Ammonium zu Nitrit und

Typ B oxidiert Nitrit zu Nitrat.

Die Verwendung von diesen definierten Mischkulturen gegenüber Reinkulturen hat den Vorteil, daß das für den Zelltyp A hemmende Nitrit laufend durch den Zelltyp B abgebaut wird. Die Symbiose der Zellen hat ebenfalls einen positiven Effekt auf die Abbauleistung und die Empfindlichkeit gegenüber Störeinflüssen.

Die eingeschlossenen und somit geschützten Zellen sind unempfindlich gegenüber pH-Wert und Temperaturschwankungen. Die Anfälligkeit gegenüber toxischen Verbindungen ist erheblich reduziert. Da die eingeschlossenen Zellen nicht von Fremdkeimen überwuchert werden können und die Hochleistungsmischkultur in dem polymeren Netzwerk wachsen kann, ohne ausgeschwemmt zu werden, kommt es zu einer Anreicherung von Nitrifikanten, wie sie beim Einsatz der Zellen in der flüssigen Phase oder auf adsorbtiven Medien nicht erreicht werden kann. Nur so ist die extrem hohe Nitrifikationsleistung dieser eingeschlossenen Zellen im Vergleich zu freien Zellen zu erklären. Darüber hinaus erklären diese Verfahrenseigenschaften auch die Tatsache, daß betriebliche Abweichung von den optimalen Bedingungen in Bezug auf pH-Wert, Temperatur oder zeitbegrenztem Anfall von toxischen Verbindungen den Prozeßablauf nicht stören.

Beispiel:

Kontinuierliche Nitrifikation in einem Abwasser eines Gichtgaswäschers (Hüttenindustrie) mit einem Ammoniakgehalt von 600 mg $NH_4$/1.

a) Klassisches Verfahren: bei einer Verweilzeit von 4 Tagen konnte ein Abbau von 600 mg $NH_4$ 1 auf 80 mg $NH_4$/1 beobachtet werden.

b) Neues Verfahren: bei einer Verweilzeit von nur 8 Stunden konnte ein Abbau von 600 mg $NH_4$/1 auf 60 mg $NH_4$ 1 beobbeobachtet werden.

Der Erfindung liegt die Aufgabe zurgrunde, ein Verfahren und eine Vorrichtung der eingangs erwähnten Art zu schaffen, die durch möglichst kurze Reaktionszeiten, sowie geringen apparativen Aufbau die Umwandlung von $NH_3$ bzw $NH_4$ zu $NO_3$ bewirkt. Gelöst wird diese Aufgabe durch die Verwendung eines Biokatalysators mit immobilisierten Zellen, die in einem Gel oder Polymer vernetzt sind oder auf andere Weise wie in den Ansprüchen 1 - 3 beschrieben, eingeschlossen sind. Durch die erfindungsgemäße Verwendung eines Biokatalysators ergibt sich eine erhöhte Biomassenkonzentration, wodurch in kürzester Zeit ein Ammoniakabbau erreicht wird. Gegenüber adsorbtiven Trägersystemen ergibt sich bzgl. der Reaktionszeit und des apparativen Aufwands eine Abkürzung der Reaktionszeit, um das 10 - 20-fache. Nach längerem Einsatz kann der Biokatalysator, falls erforderlich, ohne Schwierigkeiten ausgetauscht werden. Je nach Erfordernis kann die biologische Nitrifikation einstufig oder mehrstufig durchgeführt werden.

Die erfindungsgemäße Vorrichtung besteht aus einem oder mehreren Reaktorbehältern mit einem Biokatalysatorbett, einer Wasserzuleitung, einer Wasseraustrittsleitung sowie einer Luft- bzw. Sauerstoff- bzw. $CO_2$-Zumischleitung, mit einem Umwälzkreislauf zwischen dem Wasserauslauf und dem Wassereinlauf zum Biokatalysatorbett. Mit diesem Umwälzkreislauf kann eine pH-Wert-Einstell- und Meßvorrichtung verbunden werden, die vorzugsweise eine Base-Dosierpumpe aufweist.

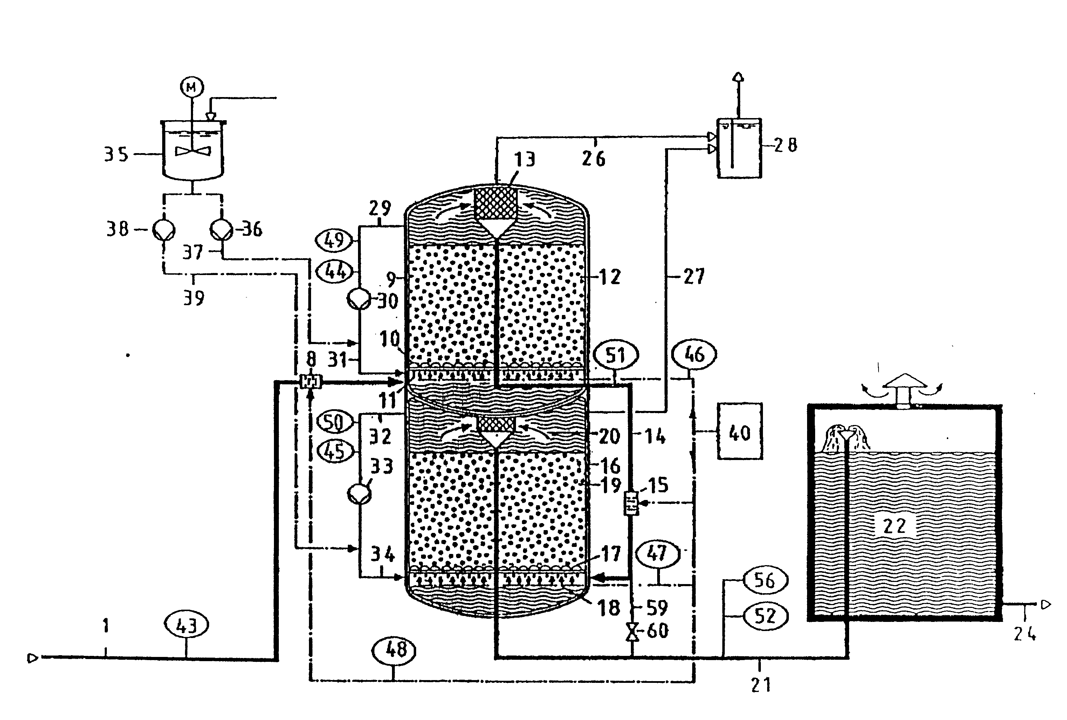

Die Erfindung wird nachstehend anhand eines in der Zeichnung schematisch dargestellten Ausführungsbeispiels näher erläutert. Das zu behandelnde Wasser gelangt über eine Zuleitung 1 und einen Mischer 8 für Sauerstoff und $CO_2$ in einen Reaktionsbehälter 9, der die erste Stufe der biologischen Nitrifikation bildet.

Im Reaktionsbehälter 9 ist im unteren Bereich eine Auflageboden 10 für ein Biokatalysatorbett 12 vorgesehen, durch den das zu nitrifizierende Rohwasser geleitet wird. Am oberen Ende des Reaktionsbehälters 9 tritt das teilweise nitrifizierte Wasser durch eine Siebvorrichtung 13 in eine Austrittsleitung 14 ein, durch die es in einen Reaktionsbehälter 16, der die zweite Stufe der biologischen Nitrifikation bildet, gelangt. Der Reaktionsbehälter 16 ist in gleicher Weise wie der Reaktionsbehälter 9 aufgebaut und weist einen Auflageboden 17 mit darüber angeordnetem Biokatalysatorbett 19 auf. Auch dieser Reaktionsbehälter 16 wird vom zu nitrifizierenden Wasser von unten nach oben durchströmt und verläßt den Reaktionsbehälter 16 über eine Siebvorrichtung 20 und eine Austrittsleitung 21. Grundsätzlich besteht auch die Möglichkeit (im Schema nicht dargestellt) durch entsprechende Anordnung der Eintrittsleitung 11 und der Austrittsleitung 14 und 21 an den Reaktionsbehältern 9 und 16 den Wasserstrom aus dem oberen Raum der Reaktionsbehälter 9 bzw. 16 in deren unteren Teil zu leiten.

Die Austrittsleitung 21 führt in einen Reinwasserbehälter 22. Über eine Leitung 24 wird das behandelte Wasser abgegeben. Zur Regelung der Nitrifikationsrate sind beide Nitrifikationsstufen mit einem eigenen regelbaren Umlaufsystem ausgerüstet. Das Umlaufsystem des Reaktionsbehälters 9 besteht aus einer Leitung 29 stromabwärts oder stromaufwärts vom Biokatalysator 12, einer Umwälzpumpe 30 und einer unterhalb des Auflagebodens 10 mündenden Leitung 31. Das Umlaufsystem für den Reaktionsbehälter 16 besteht entsprechend aus einer Leitung 32, einer Umwälzpumpe 33 und einer Leitung 34. In jeder Leitung 29 bzw. 33 sind ein pH-Wert-Meßgerät 49, 50 sowie ein Durchflußmesser 44, 45 angeordnet. Die pH-Meßgeräte 49, 50 dienen dazu, den pH-Wert in den Reaktionsbehältern 9 und 16 einzustellen.

Diese geschieht mit Hilfe von Dosierpumpen 36, 38, die über Leitungen 37, 39 mit den Leitungen 31, 34 verbunden sind. Mit Hilfe der Dosierpumpen 36, 38 wird Base aus einem Behälter 35 entnommen und in der benötigten Menge den Reaktionsbehältern 9 und 16 zugeführt. Eine zusätzliche Überwachung des pH-Wertes des gereinigten Wassers erfolgt in der Leitung 21 mit Hilfe des ph-Wert-Meßgerätes 52.

Luft, bzw. Sauerstoff und/oder $CO_2$ werden über die Mischer 8 und 15 gegebenenfalls unter Überdruck zugesetzt. Ggfs. kann die Luft bzw. $CO_2$-Dosage auch direkt in den Reaktionsbehälter 9 über ein Verteilersystem 11 und in den Reaktionsbehälter 16 über ein Verteilersystem 18 erfolgen. In der Zuleitung 1, den Leitungen 29 und 32 und den von der Sauerstoff- und $CO_2$-Dosiereinrichtung 40 abgehenden Leitungen sind zusätzlich Durchflußmesser 43 bis 48 angeordnet, mit Hilfe derer der Durchsatz durch diese Leitungen bestimmt wird.

Zwischen der Austrittsleitung 14 aus dem Reaktionsbehälter 9 und der Austrittsleitung 21 aus dem Reaktionsbehälter 16 ist eine Umgehungsleitung 59 mit einem Ventil 60 vorgesehen, so daß ein mehr oder weniger großer Teilstrom des den ersten Reaktionsbehälter durchfließenden Wassers direkt dem Reinwassersammelbehälter 23 zugeführt werden kann, ohne den Reaktionsbehälter 16 zu durchlaufen.

Der Restgehalt an $NH_3$ bzw. $NH_4$ im gereinigten Wasser wird mit Hilfe einer Meßvorrichtung 56 bestimmt. Von den Reaktionsbehältern 9 und 16 führen Gasleitungen 26 und 27 über eine Wasservorlage 28 nach außen, durch die die Reaktionsbehälter 9 und 16 entüftet werden.

## Ansprüche

1. Verfahren zum Entfernen von $NH_3$ bzw $NH_4$ aus Abwasser, Oberflächenwasser oder Grundwasser, mit Hilfe der biologischen Nitrifikation unter aeroben Bedingungen, gekennzeichnet durch die Verwendung eines Biokatalysators mit immobilisierten Zellen, die in einem Gel oder Polymer eingeschlossen sind.

2. Verfahren nach Anspruch 1, gekennzeichnet durch die Verwendung eines Biokatalysators aud einem Kern mit immobilisierten Zellen, die in einem Gel oder Polymer eingebettet sind und mit einer zusätzlichen zellfreien, für Zellen undurchlässigen Schutzschicht, bestehend aus einem Gel oder Polymer, umschlossen sind.

3. Verfahren nach Anspruch 1, dadurch gekennzeichnet, daß sich die Zellen in Hohlkugeln befinden, deren Hülle aus einer zellfreien, für Zellen undurchlässigen Schutzschicht besteht.

4. Verfahren nach Anspruch 1, 2 oder 3, gekennzeichnet durch Verwendung von Hochleistungsmischkulturen von Nitrifikanten (Nitrosomonas, Nitrobacter usw.), die $NH_3$ bzw $NH_4$ bis zum Nitrat oxidieren.

5. Verfahren durch Anspruch 1,2,3 oder 4, dadurch gekennzeichnet daß die biologische Nitrifikation ein-oder mehrstufig durchgeführt wird.

6. Verfahren nach einem oder mehreren der Ansprüche 1-5, dadurch gekennzeichnet, daß der pH-Wert in der biologischen Nitrifikation durch Zudosierung von Base eingestellt wird.

7. Verfahren nach einem oder. mehreren der Ansprüche 1-6, dadurch gekennzeichnet, daß in der biologischen Nitrifikation $O_2$ und/oder $CO_2$ bzw ein Carbonat zudosiert werden.

8. Verfahren nach Anspruch 7, dadurch gekennzeichnet, daß die biologische Nitrifikation unter $O_2$ und oder $CO_2$-Überdruck durchgeführt wird.

9. Vorrichtung zur Durchführung des Verfahrens nach Anspruch 1, 2 oder 3 bestehend aus einem Biokatalysatorbett, einer Wasserzuleitung und einer Wasseraustrittsleitung sowie einer $O_2$ und/ oder $CO_2$-Zumischleitung, dadurch gekennzeichnet, daß zwischen dem Wasserauslauf und dem Wassereinlauf ein Umwälzkreislauf (29, 30, 31 bzw. 32, 33, 34) angeordnet ist.

10. Vorrichtung nach Anspruch 9 zur Durchführung des Verfahrens nach Anspruch 5, dadurch gekennzeichnet, daß ein oder mehr in Reihe geschalteter Reaktionsbehälter betrieben werden (9 + 16).

11. Vorrichtung nach Anspruch 9 und 10 zur Durchführung des Verfahrens nach Anspruch 7, dadurch gekennzeichnet, daß die ein oder mehr in Reihe geschalteten Reaktionsbehälter unter $O_2$ und/oder $CO_2$-Überdruck betrieben werden.

12. Vorrichtung nach Anspruch 9, zur Durchführung des Verfahrens nach Anspruch 5, gekennzeichnet durch eine mit dem Umwälzkreislauf (29, 30, 31 bzw 32, 33, 34) verbundene pH-Wert-Einstell-und Meßvorrichtung (35, 36, 37, 49 bzw. 35, 38, 39, 50)

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | CHEMICAL ABSTRACTS, Band 105, Nr. 10, 8. September 1986, Seite 362, Zusammenfassung Nr. 84513s, Columbus, Ohio, US; J. TRAMPER: "Nitrification and denitrification by immobilized bacteria", & EUR. CONGR. BIOTECHNOL., 3rd 1984 (PUB. 1985), 4, 363-8 --- | 1-5 | C 02 F  3/10<br>C 02 F  3/06 |
| Y | CHEMICAL ABSTRACTS, Band 105, Nr. 10, 8. September 1986, Seiten 362,363, Zusammenfassung Nr. 84521t, Columbus, Ohio, US; H. NAKAMURA et al.: "Nitrification-denitrification of wastewater by immobilized microbial cells", & KOGAI TO TAISAKU 1986, 22(2), 135-8 --- | 1-5 | |
| Y | EP-A-0 073 675  (CORNING GLASS WORKS) * Titelseite; Zusammenfassung; Seite 7, Zeilen 9-30; Seite 11, Zeilen 12-31; Seite 12, Zeilen 15-22 * --- | 1,2,9, 10 | |
| Y | EP-A-0 173 915  (J. KLEIN et al.) * Anspruch 1 * --- | 1-5 | RECHERCHIERTE SACHGEBIETE (Int. Cl.4)<br><br>C 02 F |
| A | R. HELMER et al.: "Weitergehende Abwasserreinigung", 1977, Seiten 41-45,60, Deutscher Fachschriften-Verlag, Mainz, DE; 2.2.1. "Nitrifikationsvorgänge" * Seite 41, Absatz "pH-Wert" - Seite 45, Absatz 1; Seite 44, Absatz 3; Seite 60, Zeilen 28-31 * --- | 6,7,8 | |
| A | FR-A-2 390 192  (DEGREMONT) * Seite 9, Anspruch 1; Seite 5, Zeilen 2-5; Seite 7, Zeilen 3,4 * ---                      -/- | 7-11 | |

Der vorliegende Recherchenbericht wurde für alle Patentansprüche erstellt

| Recherchenort | Abschlußdatum der Recherche | Prüfer |
|---|---|---|
| DEN HAAG | 07-03-1988 | TEPLY J. |

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | EP-A-0 204 273  (NOELL)<br>* Spalte 6, Ansprüche 1,2,7,8,10 *<br>----- | 1,2,9,<br>10,12 | |
| | | | **RECHERCHIERTE SACHGEBIETE (Int. Cl.4)** |

Der vorliegende Recherchenbericht wurde für alle Patentansprüche erstellt

| Recherchenort | Abschlußdatum der Recherche | Prüfer |
|---|---|---|
| DEN HAAG | 07-03-1988 | TEPLY J. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
    ............................................................................
& : Mitglied der gleichen Patentfamilie, übereinstimmendes
    Dokument